# EUROPEAN PATENT SPECIFICATION

(11) **EP 1 663 134 B1**
(45) Date of publication and mention of the grant of the patent: **31.03.2010**
(21) Application number: 04780974.4
(22) Date of filing: 13.08.2004
(51) Int. Cl.: A61K 8/22, A61K 8/81, A61K 8/25, A61K 8/90, A61Q 11/00

(54) **NON-AQUEOUS LIQUID TOOTH WHITENING COMPOSITION**
NICHTWÄSSRIGE FLÜSSIGE ZAHNBLEICHZUSAMMENSETZUNG
COMPOSITION NON AQUEUSE LIQUIDE DE BLANCHIMENT DES DENTS

(30) Priority: 15.08.2003 US 641961
(43) Date of publication of application: 07.06.2006
(60) Divisional application: 10151632.6
(73) Proprietor: Colgate-Palmolive Company, New York NY 10022-7499 (US)
(72) Inventor: FEI, Lin, Kendall Park, New Jersey 08824 (US); CHOPRA, Suman, K., Dayton, New Jersey 08810 (US); MANDADI, Prakasarao, Flemington, New Jersey 08822 (US); PATEL, Neeta, Monmouth Junction, New Jersey 08852 (US); SHASTRY, Ramachandra, South Brunswick, New Jersey 08810 (US); MIRAJKAR, Yelloji-Rao, K., Piscataway, New Jersey 08854 (US); PRENCIPE, Michael, West Windsor, New Jersey 08550 (US)
(74) Representative: Jenkins, Peter David
(86) International application number: PCT/US2004/026217
(87) International publication number: WO 2005/018591

(56) References cited:
- WO-A-00/19971
- WO-A-91/07184
- WO-A-97/11676
- US-A- 3 951 838
- US-A- 4 971 782
- US-A- 5 374 368
- US-A1- 2001 021 374
- US-B1- 6 306 370

## Description

### BACKGROUND OF THE INVENTION

### 1. Field of the Invention

This invention relates to stable nonaqueous liquid tooth whitening composition.

### 2. The Prior Art

It has become desirable for a person's teeth to appear bright or "white". Society places a high value on the "whiteness" of one's teeth. One whose teeth are white may enjoy more personal confidence and satisfaction and may even enjoy greater social acceptance.

A tooth is comprised of an inner dentin layer and an outer hard enamel layer that is the protective layer of the tooth. The enamel layer of a tooth is naturally an opaque white or slightly off-white color. It is the enamel layer that can become stained or discolored. The enamel layer of a tooth is composed of hydroxyapatite mineral crystals that create a somewhat porous surface. These hydroxyapatite crystals form microscopic hexagonal rods or prisms that make up the enamel surface. As a result, the surface of the enamel layer presents microscopic spaces or pores between the prisms. It is believed that this porous nature of the enamel layer is what allows staining agents and discoloring substances to permeate the enamel and discolor the tooth. These remaining substances can occupy the microscopic spaces and eventually alter the color of the tooth.

Many substances that a person confronts or comes in contact with on a daily basis can "stain" or reduce the "whiteness" of one's teeth. In particular, the foods, tobacco products and fluids that one consumes tend to stain one's teeth. These products or substances tend to accumulate on the enamel layer of the tooth and form a pellicle film over the teeth. These staining and discoloring substances can then permeate the enamel layer. This problem occurs gradually over many years, but imparts a noticeable discoloration of the enamel of one's teeth. So long as the discolored teeth are still healthy and do not pose any health risk or problem, a product or substance that would whiten the discolored teeth would be advantageous.

It is also essential that a tooth whitening product that is to be used at home or in private by the consumer be safe and easy to use and be stable and retain its whitening efficacy during its storage on retail store shelves as well as over the period of use by the consumer.

Products and substances that are presently available to whiten teeth include a variety of different ingredients, but the primary active ingredient is a peroxide agent formulated into an aqueous liquid, solution, paste or gel. These products upon storage lose their whitening efficacy over time as peroxide compounds in aqueous solutions are relatively unstable. This tendency toward instability of peroxide has limited the utility of aqueous liquid whitening products for whitening teeth. It would be highly desirable, therefore, to provide a stable peroxide whitening liquid to effect substantive whitening.

US-A-2001/0021374 discloses took whitening compositions including a low molecular weight compound having a high acetyl group functionality useful in the production of a peroxy acid which their acts as a whitening agent.

### SUMMARY OF THE INVENTION

In accordance with this invention there is provided a homogeneous stable non-aqueous liquid tooth whitening composition according to claim 1. When applied to the teeth the composition in sufficiently viscous to form an adherent, continuous layer of the peroxide containing composition on dental enamel surfaces.

The anhydrous liquid hydrophilic polymer of the present invention provides a stable vehicle that prevents the decomposition of the peroxide whitening agent during storage and before use. Once applied on tooth surface, the saliva on the tooth enamel surface to which the composition is applied will either dissolve or disintegrate the peroxide containing layer resulting in a rapid decomposition of the peroxide, which in turn provides the whitening effect.

The non-aqueous liquid whitening composition of the present invention is a portable oral care tooth whitener that can be conveniently painted onto the tooth enamel surface. Upon the paint-on application to the teeth, the applied nonaqueous liquid whitening composition forms an adherent layer of peroxide containing suspension that has the capacity to release the peroxide whitening agent over an extended period of time, e.g., from about 5 to about 30 minutes. The applied layer adheres to the tooth surface whereby the released peroxide source then whitens the teeth to which the composition is applied. The maintenance of the composition in a non-aqueous state provides a peroxide source that is substantially stable to decomposition on storage.

### DESCRIPTION OF THE PREFERRED EMBODIMENTS

The term "hydrophilic" polymer as employed herein refers to an organic polymer which has a water solubility of at least about one gram per 100 grams of water at 25°C. The term "hydrophobic" polymer or "water-insoluble" polymer as employed herein refers to an organic polymer which has a water solubility of less than about one gram per 100 grams of water at 25°C. The term "non-aqueous" as used herein means the presence of less than 10% by weight water in the composition of the present invention.

The composition of the present invention is a viscous suspension which maintains its consistency during storage enabling the product to be painted on the tooth surface with a soft applicator brush or applied from a coating layer on a strip.

### Non-Aqueous Hydrophilic Liquid Polymer

In accordance with the invention the non-aqueous hydrophilic liquid polymer employed as the vehicle for the peroxide is polyethylene glycol.

Nonaqueous hydrophilic polymers useful in the practice of the present invention preferably provide a viscosity for the composition in the range between about 10,000 cps to 600,000 cps.

The compositions of the the present invention include as polyethylene glycols, nonionic polymers of ethylene oxide having the general formula:

HOCH₂(CH₂OCH₂)ₙO

wherein n represents the average number of oxyethylene groups. Polyethylene glycols available from Dow Chemical are designated by a number such as 200, 300, 400, 600, 2000 which represents the approximate average molecular weight of the polymer. Polyethylene glycols 200, 300, 400 and 600 are clear viscous liquids at room temperature, and are preferred for use in the practice of the present invention.

The polyethyleneglycol vehicle employed in the compositions of the invention of present in an amount of from about 62.5 to 85% by weight.

### Adhesion Enhancing Agents

Adhesion enhancing agents are used in the compositions of the present invention to enhance the adhesive properties of the anhydrous hydrophilic polymers and include inorganic materials as well as organic natural and synthetic polymers. The adhesion enhancing agent comprises 0.1 to 1 at %, based on the weight of the composition, of a polyvinyl pyrrolidone/vinyl acetate copolymer. Inorganic materials include amorphous silica compounds which function as thickening agents include colloidal silica compounds available under trademarks such as Cab-o-sil fumed silica manufactured by Cabot Corporation and distributed by Lenape Chemical, bound Brook, NJ; Zeodent 165 from J.M. Huber Chemicals Division, Havre de Grace, MD 21078; and Sylox 15 also known as Sylodent 15, available from Davison Chemical Division of W.R. Grace Corporation, Baltimore, MD 21203.

A preferred amorphous, fumed silica compound is obtainable in powder form from the Degussa Company under the trade designation "Aerosil". Various types of Aerosils are available depending on the variations in the manufacturing process. Aerosil 200 is a hydrophilic fumed silica, having a surface area of about 200 square meters per gram, an average particle size of about 10-12 nanometers and a compacted apparent density of about 50 g/l and is preferred for use in the practice of the present invention.

Organic polymers useful as adhesion enhancing agents useful in the practice of the present invention include hydrophilic polymers such as Carbomers such as carboxymethylene polymers such as acrylic acid polymers, and acrylic acid copolymers. Carboxypolymethylene is a slightly acidic vinyl polymer with active carboxyl groups. A carboxypolymethylene preferred for use in the practice of the present invention is a copolymer of acrylic acid cross linked with approximately 0.75% to approximately 1.5% polyallyl sucrose that is sold under the trade designation Carbopol 934, 974 by B.F. Goodrich.

Hydrophobic organic materials are also useful as adhesion enhancing agents in the practice of the present invention include hydrophobic materials such as waxes such as bees wax, mineral oil, plastigel, (a blend of mineral oil and polyethylene), petrolatum, white petrolatum, versagel (blend of liquid paraffin, butene/ethylene/styrene hydrogenated copolymer) acrylate and vinyl acetate polymers and copolymers, polyethylene waxes, silicone polymers such as dimethicone, silicone elastomers, organosiloxane resins and silicone gums.

Small amounts of an anhydrous solvent such as ethanol as well as humectants such as glycerin may also be used to adjust the viscosity of the liquid compositions of the present invention and are present in the nonaqueous liquid whitening compositions of the present invention in amounts of about 0.1 to about 25% by weight and preferably about 0.3 to about 20% by weight.

### Anhydrous Whitening Agent

The anhydrous whitening agent useful in the practice of the present invention is a PVP-H₂O₂ complex (hereinafter "PVP-H₂O₂ "). PVP-H₂O₂ both linear and cross linked complexes are known to the art and are disclosed in US 3,376,110 and US 3,480,557 and have been used in compositions for treating acne vulgaris (US5,122,370). PVP-H₂O₂ complexes are disclosed in US 5,122,370. PVP-H₂O₂ is stable in an anhydrous environment. By exposure to aqueous environments, as in the oral cavity, the whitening agent dissociates into individual species (PVP polymer and H₂O₂). The PVP-H₂O₂ complex is generally comprised of about 80% by weight polyvinyl pyrrolidone and 20% by weight H₂O₂.

The PVP-H₂O₂ complex is present in the liquid whitening compositions of the present invention at a concentration of from 10 to 30 % by weight.

### Surfactant

Nonionic surfactants which are compatible with peroxide compounds serve as a solubilizing, dispersing, emulsifing and wetting agents and are especially effective to solubilize a flavor if included in the liquid whitening composition. A particularly useful nonionic surfactant is a water soluble polyoxyethylene monoester of sorbitol with a C10 to C18 fatty acid, marketed commercial under the Tween trademark. The Tween surfactants are mixtures of C10 to C18 fatty acid esters of sorbitol (and sorbitol anhydrides), consisting predominately of the monoester, condensed with about 10-30, preferably about 20, moles of ethyleneoxide. The fatty acid (aliphatic hydrocarbonyl monocarboxylic acid) may be saturated or unsaturated, e.g., lauric, palmitic, stearic, oleic acids. Polysorbate 20 (e.g., Tween 20) is especially preferred and is commonly referred to as polyoxyethylene (20) sorbitan monolaurate. The nonionic surfactant constitutes about 0 to 50% by weight and preferably 0.5 to 40% by weight of the liquid composition.

### Flavor

The nonaqueous liquid whitening composition of the present invention may also contain a flavoring agent. Flavoring agents that are used in the practice of the present invention include essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint, spearmint and wintergreen. The flavoring agent is incorporated in the whitening liquid composition of the present invention at a concentration of about 0.1 to about 2% by weight and preferably about 0.1 to about 0.5% by weight.

A sweetening material is also employed as a complement to the flavoring material. Suitable sweetening agents are water soluble and include sodium saccharin, sodium cyclamate, xylitol, perillartien, D-tryptophan, aspartame, dihydrochalcones and the like, in concentrations of about 0.01 to about 1% by weight. Sodium saccharin is preferred.

The nonaqueous liquid whitening composition of the present invention is prepared in the form of a flowable viscous liquid suspension containing the whitening agent and is applied as such to the users teeth as by painting the teeth with a soft applicator brush. Application by the user, leaves a coating of the thick liquid suspension on the teeth. Contact with saliva causes the slow release of H₂O₂ to the applied tooth site from the anhydrous whitening compound providing prolonged whitening treatment of the tooth sites.

The layer of liquid peroxide containing suspension contains no ingredients imparting thereto an unacceptable taste or texture, rendering it unpleasant to the user and adheres strongly to tooth enamel. The suspension is sufficiently viscous and adherent enough to remain on the teeth for a period of time, for example about 5 to about 30 minutes to effect a whitening result and will resist the forces commonly applied by the lips and tongue. While the layer of applied liquid whitening composition is in place, the user is to refrain from mastication. The whitening composition can be removed as and when required, at will, by an employment of standard oral hygiene procedures such as brushing or by rinsing with an alcoholic mouthwash. While in place the coating releases agents contained therein at a slow, relatively constant rate and in concentration sufficient effectively to effect stain removal from the teeth.

### Peroxide Activator

Peroxide decomposition activators such as sodium bicarbonate, sodium carbonate, manganese gluconate may be incorporated in the liquid whitening gel composition of the present invention. The activator is relatively nonactive with the peroxide whitening agent when present in the stored nonaqueous liquid composition due to the anhydrous compositions. The activator functions to react with the peroxide to release oxygen when the liquid whitening composition applied to the teeth is contacted with saliva in the oral cavity. The peroxide activator is present in the non-aqueous liquid composition of the present invention at a concentration of about 0 to about 10 % by weight and preferably about 1 to about 5 % by weight.

Other ingredients which may be incorporated in the liquid whitening composition of the present invention include any of the materials commonly used in the oral care formulations. These include: antimicrobial agents, e.g., Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol); antiinflammatory agents such as ibuprofen, flurbiprofen, aspirin, indomethacine etc.; anticaries agents such as sodium-, calcium-, magnesium- and stannous fluoride, aminefluorides, disodium monofluorophosphate and sodium trimeta phosphate; plaque buffers such as urea, calcium lactate, calcium glycerophosphate and strontium polyacrylates; vitamins such as Vitamin C; plant extracts; desensitizing agents, e.g., potassium citrate, potassium chloride, potassium tartrate, potassium bicarbonate, potassium oxalate, potassium nitrate and strontium salts; agents effective against dental calculus such as pyrophosphate salts including the mono, di, tri and tetra alkali metal and ammonium pyrophosphate and tripolyphosphate salts; biomolecules, e.g., bacteriocins, antibodies, enzymes such as papain, glucoamylase; opacifying agents, pigments, coloring agents and fluoride ion providing salts having anticaries efficacy such as sodium fluoride, potassium fluoride, a tin fluoride such as stannous fluoride.

### Composition Preparation

The nonaqueous liquid whitening compositions of the present invention are prepared by adding and mixing the ingredients of the composition in a suitable vessel such as a stainless steel tank provided with a mixer. In the preparation of the liquid whitening composition, the ingredients are advantageously added to the mixer in the following order: hydrophilic polymer, anhydrous peroxide compound whitening agent, adhesive enhancer, peroxide activation and any desired flavoring or sweetener. The ingredients are then mixed to form a homogeneous dispersion/solution.

The present invention is illustrated by the following examples but is not to be limited thereby.

### Comparative Example

A series of liquid whitening paint-on compositions not according to the invention were prepared using the ingredients listed in Table I below.

| **TABLE I** | | | | | | | |
|---|---|---|---|---|---|---|---|
| | **Wt.%** | | | | | | |
| **Composition** | **A** | **B** | **C** | **D** | **E** | **F** | **G** |
| **Ingredient** | | | | | | | |
| PEG 400 | 71.20 | 33.50 | 51.50 | 21.50 | 12.50 | 37.00 | -- |
| Polyethylene/Polypropylene block copolymer* | -- | --- | -- | 50.00 | 60.00 | 35.00 | 54.5 |
| Amorphous silica** | 3.00 | 1.50 | 3.00 | 1.50 | 0.50 | 3.00 | -- |
| Sodium percarbonate | 24.00 | 24.00 | 24.00 | 25.00 | 25.00 | 24.00 | 24.00 |
| Vinyl acetate copolymer | 0.50 | -- | -- | -- | -- | -- | -- |
| Anhydrous ethyl alcohol | 0.50 | -- | -- | 0.50 | 0.50 | -- | -- |
| Polyoxyethylene sorbitan*** | -- | 40.00 | -- | -- | -- | -- | -- |
| Dimethicone (350 cst) | -- | -- | -- | -- | -- | -- | 20.00 |
| PVP/VA copolymer | -- | -- | 20.00 | -- | -- | -- | -- |
| Petrolatum | -- | -- | -- | -- | -- | -- | -- |
| Na saccharin | 0.80 | 1.00 | 1.00 | 1.00 | 1.00 | 1.00 | 1.00 |
| Flavor | -- | -- | 0.50 | 0.50 | 0.50 | -- | 0.5 |

| | | | | | | | |
|---|---|---|---|---|---|---|---|
| * Pluraflo L-1220 ****** Aerosil 200 *** Polysorbate 60 | | | | | | | |

### Stability

The shelf stability of the nonaqueous liquid whitening composition of Table I was determined by packaging the composition in sealed plastic bottles and exposing the bottles to 49°C (120°F). The percent hydrogen peroxide recovered from the liquid whitening composition after a 4 week exposure to temperatures of 49°C (120°F) determined using Iodometric Titration. The peroxide recovery results indicated that the nonaqueous liquid whitening compositions to be efficacious whitening compositions even after 4 weeks of storage at the elevated temperature of 49°C (120°F)

The whitening efficacy of the liquid compositions was determined using a duplicate pair of flow cells designed to accommodate a total of eight bovine enamel blocks (four in each cell). The bovine enamel blocks were obtained freshly stained using an established staining protocol (Indiana University, Indianapolis, IN). The initial L*, a* and b* values were matched as closely as possible prior to the experiment using a chromameter (Minolta CR-321) based on initial L*, a* and b* values (CIELAB). These initial values were typically L* = 25.00, a* = 3.00, and b* = 5.00 to L* = 35.00, a* = 5.00, and b* = 7.00. The L, a, b values were measured four times at slightly differing locations on the surface of the bovine enamel blocks.

To simulate the saliva of the human mouth, an artificial saliva buffer solution maintained at 37°C was prepared which contained the salts usually present in saliva at levels typical to the levels found in human saliva.

The bovine enamel blocks were placed in the flow cells and the liquid compositions evenly applied using a brush, the amount of product applied being determined using the weight difference of the container. Flow over the teeth was 0.6 ml/min. for 30 min. Average initial and final chromometer readings were used to calculate ΔE according to ΔE = ((L_{f}- Lᵢ)² + (b_{f}-bᵢ)² + (a_{f}-aᵢ)²)^{½}. The final ΔE reported was the average over all observations after the rejection of outliers using the Student's test (95% confidence level). The results are recorded in Table II below. For purposes of comparison, the whitening efficacy test procedure of the comparative examples I was repeated with the exception that a commercially available aqueous paint-on tooth whitening composition designated composition X containing 6.75% by weight hydrogen peroxide was also evaluated for whitening efficacy. The results of these evaluation tests are recorded in Table **II** below.

| **TABLE II** | |
|---|---|
| **Whitening Efficacy** | |
| **Liquid Gel Formula** | **ΔE** |
| A | 17 |
| B | 17 |
| C | 17 |
| D | 34 |
| E | 20 |
| F | 26 |
| G | 26 |
| X | 5.3 |
| Y | 5.3 |

The results recorded in Table II indicate that the whitening efficacy (ΔE) of the compositions (Compositions A-G) are substantially more efficacious than the comparative commercially available liquid tooth whitening compositions X and Y.

### Example

A series of anhydrous liquid whitening gel compositions designated "Compositions I-M" were prepared with PVP-H₂O₂ in a nonaqueous liquid vehicle in which a polyvinyl pyrolidone/vinyl acetate copolymer (PVP/VA) or fumed silica was the rheology modifier. The ingredients of these compositions are listed in Table III below.

| **TABLE III** | | | | | |
|---|---|---|---|---|---|
| **Composition** | **I (Wt.%)** | **J (Wt.%)** | **K (Wt.%)** | **L (Wt.%)** | **M (Wt.%)** |
| **Ingredients** | | | | | |
| Na percarbonate | -- | -- | -- | -- | 2 |
| PVP-hydrogen peroxide | 30 | 30 | 20 | 10 | 30 |
| PEG 600 | 62.9 | -- | 73 | 85 | 64 |
| PEG 400 | -- | 62.5 | -- | -- | -- |
| Fumed silica | 5 | 5 | 5 | 3 | 3 |
| NaHCO₃ | 2 | 2 | 1 | -- | -- |
| NaCO₃ | -- | -- | -- | 1 | -- |
| PVP/VA polymer | 0.1 | 0.5 | 1 | 1 | 1 |
| **Total** | **100.00** | **100.00** | **100.00** | **100.00** | **100.00** |

### Preparation of Compositions I-M

The polyethylene glycols (PEG) were added to PVPH₂O₂ with gentle mixing to form a slurry. The mixture was then heated to about 70°C with continuous stirring until the PVPH₂O₂ was fully dissolved in the PEG mixture. Then PVPNA polymer is added to the PEG vehicle followed by the addition of fumed silica. The mixture was homogenized until a homogenous mixture was formed (about 10 minutes). Solid activator (NaHCO₃) was added to the mixture at the last stage, and stirred uniformly, dispense the activator.

The whitening efficacy of the liquid whitening gel formulations I and J was determined using bovine teeth following the procedure of the comparative example.

| **TABLE IV** | | |
|---|---|---|
| **Whitening Efficacy** | | |
| **Liquid Gel Formula** | | **ΔE** |
| I | | 20 |
| J | | 20 |

## Claims

1. A homogeneous stable, non-aqueous liquid tooth whitening composition suitable for application to a surface of a tooth in the oral cavity comprising
a) an anhydrous peroxide compound dispersed in an orally acceptable anhydrous hydrophilic liquid polymer, wherein the anhydrous hydrophilic liquid polymer is polyethylene glycol which comprises 62.5 to 85wt%, based on the weight of the composition, and
b) an adhesion enhancing agent,
which composition when applied topically to the tooth surface forms an adherent layer containing the peroxide compound, wherein said peroxide compound is thereafter released from the layer to effect tooth whitening,
**characterised in that** the anhydrous peroxide compound is PVP/H₂O₂, the concentration of PVP/H₂O₂ present in the composition is from 10 weight % to 30 weight %, and the adhesion enhancing agent comprises 0.1 to 1 wt%, based on the weight of the composition, of a polyvinyl pyrrolidone/vinyl acetate copolymer.

## Patentansprüche

1. Homogene, stabile, nicht-wässrige, flüssige Zahnbleichmittel-Zusammensetzung, die zur Anwendung auf eine Zahnoberfläche in der Mundhöhle geeignet ist und
a) eine wasserfreie Peroxidverbindung, die in einem oral annehmbaren, wasserfreien, hydrophilen, flüssigen Polymer dispergiert ist, wobei das wasserfreie, hydrophile, flüssige Polymer Polyethlenglykol ist, das 62,5 bis 85 Gew.% auf Basis des Gewichts der Zusammensetzung umfasst, und
b) ein adhäsionsverbesserndes Mittel umfasst,
wobei die Zusammensetzung bei der topischen Auftragung auf die Zahnoberfläche eine anhaftende, die Peroxidverbindung enthaltende Schicht bildet, wobei die Peroxidverbindung danach von der Schicht zur Zahnbleichung freigesetzt wird,
**dadurch gekennzeichnet, dass** die wasserfreie Peroxidverbindung PVP/H₂O₂ ist, die Konzentration von in der Zusammensetzung vorhandenem PVP/H₂O₂ 10 Gew.% bis 30 Gew.% ist und das adhäsionsverbessernde Mittel 0,1 bis 1 Gew.%, auf Basis des Gewichts der Zusammensetzung, eines Polyvinylpyrrolidon/Vinylacetat-Copolymers umfasst.

## Revendications

1. Composition liquide non aqueuse de blanchiment des dents homogène et stable appropriée pour une application à une surface d'une dent dans la cavité orale comprenant
a) un composé peroxyde anhydre dispersé dans un polymère liquide hydrophile anhydre oralement acceptable, où le polymère liquide hydrophile anhydre est du polyéthylène glycol qui comprend 62,5 % à 85 % en poids, par rapport au poids de la composition et
b) un agent améliorant l'adhérence,
laquelle composition quand elle est appliquée de manière topique à la surface des dents forme une couche adhérente contenant le composé peroxyde, où ledit composé peroxyde est ensuite libéré à partir de la couche pour réaliser un blanchiment des dents,
**caractérisée en ce que** le composé peroxyde anhydre est PVP/H₂O₂, la concentration de PVP/H₂O₂ présent dans la composition est de 10 % en poids à 30 % en poids, et l'agent améliorant l'adhérence comprend 0,1 % à 1 % en poids, par rapport au poids de la composition, d'un copolymère polyvinylpyrrolidone/acétate de vinyle.
